# EUROPEAN PATENT SPECIFICATION

(11) **EP 1 572 675 B1**
(45) Date of publication and mention of the grant of the patent: **09.05.2007**
(21) Application number: 03708418.3
(22) Date of filing: 30.03.2003
(51) Int. Cl.: C07D 311/62, A61K 31/35, A61P 35/00

(54) **NEW ALPHA-GLUCOSIDASE INHIBITORS AND THEIR SYNTHESIS FROM A NATURAL SOURCE**
ALFA-GLYCOSIDASE INHIBITOREN UND DEREN SYNTHESE AUS EINER NATÜRLICHEN QUELLE
NOUVEAUX INHIBITEURS DE L'ALPHA-GLUCOSIDASE ET LEUR SYNTHESE A PARTIR D'UNE SOURCE NATURELLE

(30) Priority: 17.12.2002 WO PCT/IB02/05426
(43) Date of publication of application: 14.09.2005
(73) Proprietor: COUNCIL OF SCIENTIFIC AND INDUSTRIAL RESEARCH, New Delhi 100 001 (IN)
(72) Inventor: RAO, Janaswamy Madhusudana, 500 007 Andhra Pradesh (IN); RAO, Rao Jagadeeshwar, 500 007 Andhra Pradesh (IN); KUMAR, Upparapalli Sampath, 500 007 Andhra Pradesh (IN); REDDY, Singireddy Venkat, 500 007 Andhra Pradesh (IN); TIWARI, Ashok Kumar, 500 007 Andhra Pradesh (IN); YADAV, Jhillusingh, 500 007 Andhra Pradesh (IN); RAGHAVAN, Kondapuram Vijaya, 500 007 Andhra Pradesh (IN)
(74) Representative: Ekström, Nils
(86) International application number: PCT/IB2003/001157
(87) International publication number: WO 2004/054995

(56) References cited:
- WO-A-99/19319
- D.A. YOUNG ET AL.: "SYNTHESIS OF CONDENSED TANNINS.PART19." JOURNAL OF THE CHEMICAL SOCIETY, PERKIN TRANSACTIONS 1., vol. 11, 1987, pages 2345-51, XP002251386 CHEMICAL SOCIETY. LETCHWORTH., GB ISSN: 0300-922X
- C. RAMESH: "A MILD,HIGHLY SELECTIVE A. REMARKABLY EASY PROCEDURE FOR DEPROTECTION OF AROMATIC ACETATES" TETRAHEDRON., vol. 59, no. 7, 10 February 2003 (2003-02-10), pages 1049-54, XP004406418 ELSEVIER SCIENCE PUBLISHERS, AMSTERDAM., NL ISSN: 0040-4020

## Description

### Field of invention:

This invention relates to the identification of novel α-glucosidase inhibitory compound (-)-mesquitol isolated in significant yield from traditional medicinal plant *Dichrostachys cinerea* and further modification of (-)-mesquitol to enhance the α-glucosidase inhibitory potential. This invention also identifies the usage of (-)-mesquitol and its analogues, based on their α-glucosidase inhibitory activity, as broad based potential therapeutics as antihyperglycemic, antidiabatic, antiobesity, antiviral, anticancer, immunestimulants and the like.

### Background of the invention:

It is described that an α-glucosidase inhibitors inhibits the α-glucosidase localized at the fine villies in the small intestine, and controls the rapid increase in blood glucose after meal and next increase in blood insulin level (*Diabetes Medicine,* 10, 688, 1993). Since they suppresses / slow down the metabolism of dietary carbohydrates in human and animals, and exhibit an inhibitory effect of blood glucose increases, they are found effective in improving hyperglycemic conditions as well as various diseases induced by hyperglycemia such as obesity and diabetes.

Glucosidases are also found involved in the transformation of normal cells to cancer cells and in tumor cell invasion and migration. It has also been observed that level of serum glucosidases are elevated in many patients with different tumors and are thought to be involved in the degradation of the extra cellular matrix in tumor cell invasion *(Cancer Matastasis Rev.4,* 81, 1985). Therefore, the use of glucosidase inhibitors to prevent abberations during glycoprotein processing and to inhibit catabolic glycosidases is being actively pursued as therapeutic strategy for cancer. (*Phytochemistry* 56, 265, 2001).

Furthermore, many animal viruses contain an outer envelop, which is composed of one or more viral glycoprotein. These glycoproteins are often essential proteins in that they are required in the viral life cycle, either in viron assembly and secretion and /or infectivity. As the processing of these glycoproteins occurs through the cellular machinery, inhibitors of processing α-glucosidases have been shown to decrease the infectivity of broad range of human pathogenic viruses (*FEBS letters* 430, 17, 1998 and *Phytochemistry* 56, 265, 2001).

Similarly, α-glucosidase inhibitors have also been found to restore the immune response of immunocompromized experimental animals (*Chem. Pharm. Bull.* 39, 2807, 1991). Therefore α-glucosidase inhibitors are also extensively been worked out for their exploitation as immunostimulants.

There are several α-glucosidase inhibitors known in the literature and are under clinical practice (*Phytochemistry* 56, 265, 2001). However, it is still felt that when more of the α-glucosidase inhibitor compounds of natural origin with varied skeleton become available commercially, there is sure to be even wider range of potentially valuable activities found than shown to date.

Plants continue to be used world wide for the treatment of disease and novel drug entities continue to be developed through research in to their constituents. Despite the massive arsenal of clinical agents developed by the pharmaceutical industry there has been aversion by many members of the public. This public aversion further paved the way for use of herbal medicines. Therefore, herbal remedies have proved to be popular as alternative treatment of disease. Due to this powerful ***Green Wave*** sweeping all over the world, the demand for herbal drug has increased several folds.

This current trend has accelerated the scientific investigations and evaluation of folklore and traditional medicinal plants used for the treatment of variety of ailments world over. These efforts have led to the isolation and identification of several novel chemical entities. These chemical entities were also found to possess variety of biological activities of multiple therapeutic importances.

*Dichrostachys cinerea* is a medicinal plant used in the traditional Indian system of medicine. It is widely advocated in diuretic, lithotriptic, anodyne, and inflammatory conditions. Further, it is found useful in arthralgia, elephantiasis, dyspepsia, diarrhea, nephropathy etc (*Indian Medicinal Plants,* Vol.2 p.330, 1995). *Dichrostachys cinerea* is also found useful in opthalmia, rheumatism, urinary calculi and renal troubles (*Wealth of India* Vol.3 p.56, 1952) and has been reported to possess protease inhibitory (*CA, 90,* 118086u), antifungal (*Ind. J. Plant. Physiol,* 29, 278-80, 1986), and antibacterial activity (*Fitoterapia,* 59, 57-62, 1988.).

In this invention, (-)-mesquitol is isolated from the methanolic extract of stem of *Dichrostachys cinerea* in very good yield (1.5% yield from the dried plant material). (-)-mesquitol is an optical isomer of (+)-mesquitol which was previously isolated from *Prosopis grandulosa in* 0.01% yield (*J. Chem. Soc. Perkin. Trans I,* 1737, 1986). However, this isomer of mesquitol has not tested for any biological activity to the best of our search.

The natural occurrence of a new class of condensed tannins in the heartwood of *Prosopis glandulosa* ('Mesquite') has however been demonstrated, *J. Chem. Soc., Perkin. TransI.,* 11, 2345-51, 1987. These oligomers, based on bi- and terphenyl type flavan-3-ols, presumably originate from oxidative phenol coupling of the predominant metabolite, (2R,3S)-2,3-trans-3',4',7,8-tetrahydroxyflavan-3-ol[(+)-mesquitol (2)] to give [5,6]-bis-(+)-mesquitol and similar condensation with (+)-catechin (1) [(2R,3S)-2,3-*trans*-3',4',5,7-tetrahydroxyflavan-3-ol], leading to atropisomeric [5,8]-(+)-mesquitol-(+)-catechins and [5,6:5,8]-bis-[(+)-mesquitol]-(+)-catechins. Structural elucidation of these analogues and also definition of the conformations of the atropsiomeric [5,8]-biphenyl type biflavan-3-ols, were accomplished by nuclear Overhauser effect (n.O.e.) difference spectroscopy (¹H homonuclear). During the course of structural confirmation of the dimeric homologues by synthesis *via* phenol oxidative coupling, an increased rate of crossed condensations compared with competing selfcondensation of either (+)-mesquitol (2) or (+)-catechin (1) was evident. These observations prompted detailed investigation of the oxidative dimerization of (+)-catechin and also extension of 'mixed' coupling with (+)-mesquitol aimed at formation of the [5,6:5,8]-*m-*terphenyl type triflavan-3-ols.

Despite the wide use of *Dichrostachys cinerea* in Indian traditional system of medicinal practice, efforts of isolating and identifying biologically active molecules are still lacking. As molecules isolated from tradional medicinal plants have shown multiple biological activities of therapeutic importance, we investigated *Dichrostachys cinerea* and observed that (-)-mesquitol is present in *D. cinerea* in significant yield and possess potent α-glucosidase inhibitory activity, which may find broad therapeutic application. We also made efforts to prepare analogues of the compound (-)-mesquitol in order to enhance the α-glucosidase inhibitory activity.

### Objects of the invention

The main object of the present invention is to provide (-)-mesquitol and analogues of (-)-meaquitol.

Another object of the invention is to provide semi-synthetic 3- O-alkyl or aryl estersof (-)-mesquitol.

Still another object of the invention is to provide pharmaceutical compositions comprising (-)-mesquitol or its semi-synthetic 3- O-alkyl or aryl esters to provide α-glucosidase inhibitor.

Still another object of the invention is t provide a process for the isolation of (-)-mesquitol from *Dichrostachys cinerea.*

Furthermore, the object of the invention relates to enhance the α-glucosidase inhibitory potential of the parent compound (-)-mesquitol by preparing the esters, which includes both aliphatic and aromatic.

### Summary of the Invention

Accordingly, the present invention provides novel α-glucosidase inhibitory compound (-)-mesquitol and its analogs isolated in significant yield from traditional medicinal plant *Dichrostachys cinerea* and further modification of (-)-mesquitol to enhance the α-glucosidase inhibitory potential. This invention also identifies the usage of (-)-mesquitol and its analogues, based on their α-glucosidase inhibitory activity, as broad based potential therapeutics as antihyperglycemic, antidiabatic, antiobesity, antiviral, anticancer, immunestimulants and the like

### Detailed Description of the Invention

Accordingly, the present invention provides (-)-Mesquitol and its semi-synthetic 3- O-alkyl or aryl esters represented by the general formula (4) 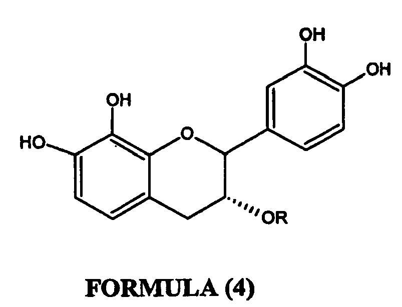 wherein, R = H, CₙH₂ₙ₊₁ (n = 1 to 16); -COC₆H₄X [wherein X = H, F, Cl, Br, I, NO₂, CN, NH₂, OR₁ {R₁ = H; CₙH₂₊₁ (n = 1 to 8)}]

In an embodiment of the present invention, the preferred compounds of general formula 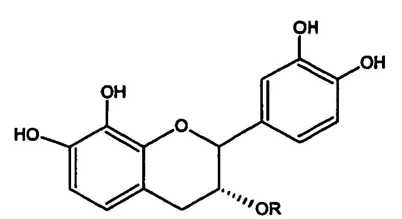 (4), where R selected is listed in the following table:

| **Serial No.** | **Compound Code** | **R** |
|---|---|---|
| 1. | 4a | Acetyl |
| 2. | 4b | Butyryl |
| 3. | 4c. | Hexanoyl |
| 4. | 4d. | Decanoyl |
| 5. | 4e | Myristoyl |
| 6. | 4f | Palmitoyl |
| 7. | 4g | Stearoyl |
| 8. | 4h | Benzoyl |
| 9. | 4i | o-chloro benzoyl |
| 10. | 4j | p-methoxy benzoyl |
| 11. | 4k | p-flouro benzoyl |

An embodiment of the invention, the said compounds exhibit α-glucosidase inhibitory activity.

In another embodiment, the α-glucosidase inhibitory activity of 3-O-aliphatic esters of (-) mesquitol increases with an increase in carbon chain length up to sixteen carbon atoms.

Still another embodiment, palmitoyl, myristoyl and decanoyl esters of (-) mesquitol are more potent α-glucosidase inhibitors than the standard drug 1-deoxy nojirimycin.

Still another embodiment, the α-glucosidase inhibitors of 3-O-aromatic esters of (-) mesquitol are better than the parent compound.

Yet another embodiment, the benzoyl and p-flourobenzyl ester of (-) mesquitol are more potent α-glucosidase inhibitor than the standard drug 1- deoxy nojirimycin.

Still yet another embodiment, the above said compound (-) mesquitol and its analogues are useful in the management and treatment of diseases like hyperglycemia, hyper insulinemia, hypolipoproteinemia, cancer, viral infection, hepatitis B and C, HIV and AIDS.
The IC₅₀ values of the compounds are in the range of 32.0 to 83.0 µm.

One more embodiment of the invention provides a pharmaceutical composition for α-glucosidase inhibitor activity, the said composition comprising administering a pharmaceutically effective dosage of (-) mesquitol or its analogues or combination thereof to the subject in need of.

Another embodiment of the invention, the composition optionally comprises pharmaceutically acceptable additives. The additive is selected from nutrients such as proteins, carbohydrates, sugars, talc, magnesium stearate, cellulose, calcium carbonate, starch-gelatin paste and/or pharmaceutically acceptable carriers, excipient, diluents or solvent.

Another embodiment of the invention, the above composition is used singly or in combination with pharmaceutically acceptable analogues.

Still another embodiment of the invention, the said composition may comprise pharmaceutically acceptable additives such as carrier, diluents, and adjuvant.

Still another embodiment, the composition may be administered systemically or orally.

Yet another embodiment, the subject are selected from animals or mammals, preferably humans.

One more embodiment of the invention provides a process for the preparation of semisynthetic 3-O-alkyl or aryl esters of (-) mesquitol, the said process comprising steps of:
a) drying the wood of Dichrostachys cinerea in shade,
b) powdering the shade dried wood of step (a),
c) extracting the powder of step (b) with petroleum ether, followed by halogenated hydrocarbon solvent to obtain the plant extracts and a plant residue,
d) soaking the plant residue of step (c) in methanol, filtering and concentrating the methanol extract to obtain a residue,
e) purifying the residue of step (d) over silica gel column by eluting with mixture of organic solvent,
f) obtaining pure (-) mesquitol,
g) adding to (-) mesquitol of step (f) anhydrous potassium carbonate, a ketonic solvent, benzyl halide and refluxed the mixture under nitrogen atmosphere for a time period of 2hour to 8hour,
h) filtering the mixture of step (g), washing the residue with ketonic solvent, combining the filtrate and the wash, evaporating under reduced pressure to obtain a residue,
i) purifying the residue of step (h) to obtain pure terra-O-benzyl-mesquitol of formula (2),
j) treating the compound of formula (2) of step (i) with N,N'-Dicyclohexyl carbodiimide (DCC) required aliphatic acid in an anhydrous methylene chloride under nitrogen atmosphere followed by addition of 4-dimethyl aminopyridine, stirring the mixture for 6h to 18 h at room temperature, filtering the mixture, washing the residue with methylene chloride, combining the filtrate and washing to obtain methylene chloride solution,
k) washing the methylene chloride solution of step (j) with water, drying over anhydrous sodium sulphate, filtered, evaporating the solvent to obtain a residue,
l) purifying the residue of step (k) to obtain the required 3-O-alkyl esters of tetra-o-benzyl (-) mesquitol of formulae 3a to 3g.
m) providing a cooled solution of compound (2) in anhydrous methylene chloride along with triethyl amine, flushing nitrogen,
n) adding required benzoyl chloride to step (m) mixture stirring the mixture for 2h to 6h at an ambient temperature,
o) adding water to the reaction mixture of step (n), extracting with methylene chloride, separating methylee chloride layer and aqueous layer,
p) drying methylene chloride layer of step (o) over anhydrous sodium sulphate, filtering and evaporating the solvent to obtain a residue,
q) purifying the residue of step (p) to obtain 3-O-aryl ester of tetra-o-benzyl (-) mesquitol of formula 3h to 3k, and
r) stirring the compound of step (1) or step (q) in an alcoholic solution in the presence of palladium charcoal and hydrogen for a time period of 4h to 8h at room temperature, and
s) filtering the mixture of step (r), removing the solvent from the filtrate to obtain the required 3-O-alkyl or aryl esters of (-) mesquitol of general formula (4).

Another embodiment of the invention, the mixture of organic solvent used for eluting, is a mixture of chloroform and methanol, wherein the mixture of solvent used is chloroform-methanol (96:4).

Another embodiment, the ketonic solvent used is selected from ethyl-methyl ketone, acetone and methyl isobutyl ketone, preferably acetone.

Still another embodiment, the aliphatic acid used is selected from a group consisting of acetic acid, butyric acid, hexanoic acid, decanoic acid, myristic acid, palmitic acid or stearic acid.

Yet another embodiment, the benzoyl chloride used is selected from a group consisting of halo benzoyl chloride, alkoxy benzoyl chloride, cyano benzoyl chloride, amino benzoyl chloride and nitro benzoyl chloride.

Yet another embodiment, the halo benzoyl chloride used is o-chlorobenzoyl chloride or p-fluoro benzoyl chloride.

Yet another embodiment, the alkoxy benzoyl chloride used is p-methoxy benzyl chloride.

Another embodiment, the 3-O-esters of (-) mesquitol obtained exhibit α-glucosidase inhibitor activity.

Yet another embodiment, the α-glucosidase inhibitory activity of 3-O-aliphatic esters of (-) mesquitol increases with an increase in carbon chain length upto sixteen carbon atoms.

Yet another embodiment, palmitoyl, myristoyl and decanoyl esters of (-) mesquitol are more potent than the standard α-glucosidase inhibitor 1-deoxy nojirimycin.

Yet another embodiment, the α-glucosidase inhibition of 3-O-aromatic esters of (-) mesquitol is better than the parent compound.

Yet another embodiment, the 3-O aromatic esters of (-) mesquitol are benzoyl, o-chlorobenzoyl, p-methoxy benzoyl or p-fluoro benzoyl esters.

Yet another embodiment, the benzoyl and p-flourobenzyl ester of (-) mesquitol are more potent than the standard α-glucosidase inhibitor 1- deoxy nojirimycin.

Yet another embodiment, the 3-O-alkyl or aryl esters of (-) mesquitol obtained are useful in the management and treatment of diseases like hyperglycemia, hyper insulinemia, hypolipoproteinemia, cancer, viral infection, hepatitis B and C, HIV and AIDS.

Yet another embodiment, the 3-O-alkyl or aryl esters of (-) mesquitol obtained has IC₅₀ value in the range of 32.0 to 83.0 µm.

In the present invention, a compound selected from the group consisting of semi-synthetic occurring compounds represented by the general formula **4, which** includes aliphatic and aromatic esters of (-)-mesquitol **(Scheme-1)** and their usage as α-glucosidase inhibitors. Where R is selected from a group consisting of acetyl, butyryl, hexanoyl, decanoyl, myrystoyl, palmitoyl and steroyl in the case of aliphatic esters. Where R₁ is selected from the group consisting of benzoyl, *p*-methoxy benzoyl, *o*-chloro benzoyl and *p*-flouro benzoyl in the case of aromatic esters.

The aliphatic esters of (-)-mesquitol **(4a-g)** are prepared by the condensation of (-)-3',4',7,8-tetra-O-benzyl mesquitol with the corresponding aliphatic acid in the presence of Dicyclohexyl carbodiimide followed by debenzylation. The aromatic esters of (-)-mesquitol **(4h-k)** are prepared by the reaction of (-)-3',4',7,8-tetra-O-benzyl mesquitol with corresponding acid chloride in the presence of triethylamine followed by debenzylation. The synthetic routes yielded the required target compounds in moderate to good yields.

The application also relates to the pharmaceutical compositions comprising an effective amount of compound according to formula **4a-g** or **4h-k** together with a pharmaceutically acceptable carrier and to methods as α-glucosidase inhibitors in management and treatment of human diseases like hyperglycemia, hyperinsulinemia, hyperlipoproteinemea, cancer, viral infection, hepatitis B and C, HIV and AIDS etc.

The present invention embodies α-glucosidase inhibitory activities to the aliphatic esters and aromatic esters of (-)-mesquitol. The α-glucosidase inhibitory potential of aliphatic esters has shown steady increase with the increase in the chain length up to a length of sixteen carbons. All the aromatic esters have shown better α-glucosidase inhibitory potential than the parent compound, (-)-mesquitol.

A method for synthesizing non-naturally occurring esters of (-)-mesquitol which includes aliphatic and aromatic. All the compounds are useful as potential α-glucosidase inhibitors. α-glucosidase inhibitors present a broad spectrum of biological activities useful for therapeutic applications as antihyperglycemic, antiviral, anti-HIV, and anti cancer and so on. The aliphatic esters are prepared by the condensation of (-)-3',4',7,8-tetra-O-benzyl mesquitol with the corresponding aliphatic acid in the presence of Dicyclohexyl carbodiimide followed by debenzylation. The aromatic esters are prepared by the reaction of (-)-3',4',7,8-tetra-O-benzyl mesquitol with corresponding acid chloride in the presence of triethylamine followed by debenzylation. The synthetic schemes yielded the required target compounds in moderate to good yields. Both the aliphatic esters and aromatic esters were tested for their α-glucosidase (yeast) inhibitory potential. The aliphatic esters have shown steady increase in their inhibitory activity with the corresponding increase in the chain length up to a length of sixteen carbons followed by decrease in the activity. All the aromatic esters showed better α-glucosidase inhibitory activity than the parent compound (-)-mesquitol.

The following examples are provided as illustration only and should not be construed to limit the scope of the present invention. Any average person skilled in the art can able to perform the invention.

### Examples

### Example 1

**A. Isolation of (-)-mesquitol (1):** The shade dried wood powder of *D.cinerea* (2 Kg) was loaded on a soxhlet apparatus, extracted with petroleum ether followed by extraction with chloroform. The residue obtained after extraction with petroleum ether and chloroform was soaked in methanol for 24 hr at room temperature. The methanol extract was filtered and concentrated under vacuum to obtain 50 g of the extract. The extract was then subjected to column chromatography using silica gel (60-120 mesh). The column is eluted with chloroform methanol gradient. The fractions eluted at 4% methanol in chloroform yielded (-)-mesquitol (30 g).

### Example 2

### B. Preparation of (-)-3',4',7,8-tetra-O-benzyl mesquitol (2):

To a mixture of (-)-mesquitol 1 (1g, 3.45mmol), anhydrous potassium carbonate (2.41g, 17.2mmol) in 20ml acetone, benzyl bromide (2.96g, 17.2mmol) was added. The mixture was refluxed under nitrogen atmosphere for 4 h. After completion of the reaction potassium carbonate was filtered washed with excess of acetone (2 x 50 ml). The combined acetone layers are concentrated under vacuum. The residue was purified by column chromatography on silica gel (60-120 mesh) to yield (-)-3',4',7,8-tetra-O-benzyl mesquitol **2** (2g) in pure form.

### Example 3

### C. Preparation of 3-O-aliphatic esters of (-)-3',4',7,8-tetra-O-benzyl mesquitol (3a-g):

The aliphatic esters **(Scheme.1)** were prepared by condensing the corresponding acids with (-)-3',4',7,8-tetra-O-benzyl mesquitol **2** in the presence of N,N'-Dicyclohexyl carbodiimide (DCC). In brief the corresponding acid (0.308mmol) and DCC (0.370mmol) were cooled and stirred in anhydrous methylene chloride (5ml) for 15 min under nitrogen atmosphere. To this mixture compound **2** (0.308mmol) in anhydrous methylene chloride (3ml) was added followed by the addition of catalytic amount of 4-Dimethylamino pyridine (0.030mmol). The entire mixture was stirred at room temperature for 12 hr under nitrogen. After completion of the reaction, the reaction mixture was filtered and washed with methylene chloride (2x10ml). The combined organic layers were washed with water (2 x 25 ml), dried over anhydrous sodium sulphate and concentrated under vacuum. The residue was purified by chromatography on silica gel (60-120mesh) to give the corresponding 3-O-esters of (-)-3',4',7,8-tetra-O-benzyl mesquitol 2 (3a-g) in excellent yields.

### Example 4

**D. Preparation of 3-O-aromatic esters of (-)-3',4',7,8-tetra-O-benzyl mesquitol (3h-k): (Scheme.1)** The (-)-3',4',7,8-tetra-O-benzyl mesquitol 2 (0.308mmol) was cooled in anhydrous methylene chloride (5ml) along with triethylamine (0.370mmol) under nitrogen atmosphere. To this mixture the acid chloride (0.370mmol) of the corresponding aromatic acid was added and stirred for 4 hr. After completion of the reaction, the reaction mixture was diluted with water, extracted with methylene chloride (2x10ml), dried over anhydrous sodium sulphate and concentrated under vacuum. The residue was purified by column chromatography on silica gel (60-120mesh) to yield the corresponding 3-O-esters of (-)-3',4',7,8-tetra-O-benzyl mesquitol **2 (3h-k)** in excellent yields.

### Example 5

### E. Preparation of 3-O-aliphatic and aromatic esters of (-)-mesquitol (4a-k):

Both the aliphatic and aromatic esters of 3',4',7,8-tetra-O-benzyl mesquitol (3a-g and **3h-k**) were hydrogenolysed using palladium on carbon under hydrogen atmosphere. In general to a solution of the ester in methanol, palladium on carbon (10 mol %) was added and the mixture was stirred under hydrogen balloon for 5 h at room temperature. The catalyst was filtered over celite washed with methanol and the methanolic solution was concentrated under reduced pressure to obtain 3-O-aliphatic and aromatic esters of (-)-mesquitol **(4a-k)**.

### Example 6

**F. Determination of α-Glucosidase inhibition activity of compounds:** The α-glucosidase inhibitory assay was done by the chromogenic method. In brief 10µl of test compounds dissolved in DMSO (5mg/ml and subsequent dilutions) were incubated for 5 min. with 50µl of yeast α-glucosidase [Sigma] enzyme prepared in 100mM phosphate buffer (pH 7.00). After 5 minutes of incubation, 50µl of 5mM substrate (p-nitrophenyl-α-D-glucopyranoside [Sigma] prepared in the same buffer) was added. The pre-substrate and 5-min post-substrate addition absorbances were recorded at 405nm spectrophotometrically. The increase in absorbance from pre-substrates addition to post substrates reaction were obtained. Percent inhibition was calculated by (1-Absorbance test/ Absorbance control) x 100 and 50% inhibitory concentration (IC50) was calculated by applying suitable regression analysis.

### The physical and spectral data of the compounds:

**1. (-)-mesquitol (1):** m.p 252°C, EIMS 290 (M⁺, ¹H NMR (200MHz, acetone- d₆) δ 7.95 (2-OH, s), 7.25, 7.55 (2-OH, each singlet), 6.88-6.72 (3H, m, H-2',5',6'), 6.40 (2H, s, H-5,6), 4.62 (1H, d, *J*=7.5Hz, H-2), 4.0 (1H, brs, OH-3), 4.0 (1H, m, H-3), 2. 89 (1H, dd, *J=5* and 15Hz, H-4eq), 2.71 (1H, dd, *J*=8 and 15.0Hz, H-4ax).
**2. (-)-3',4',7,8-tetra-O-benzyl mesquitol** (2):m.p 128°C, FABMS 651 (M⁺+H), ¹H NMR (200MHz, CDCL₃) δ 7.48-7.16 (20H, m, H-4xOCH₂Ph , 7.02 (1H, s, H-2'), 6.92 (2H, s, H-5',6'), 6.68 (1H, d, *J*=8Hz, H-5), 6.50 (1H, d, *J*=8Hz, H-6), 5.15-5.01 (8H, each s, H-4xOCH₂Ph), 4.61 (1H, d, *J*=7.2Hz, H-2), 3.90 (1H, m, H-3), 2.95 (1H, dd, *J*=5.2 and 15.5Hz, H-4eq), 2.78 (1H, dd, *J*=7.5 and 15.5, H-4ax).
**3. (-)-3-O-acetyl mesquitol (4a):** m.p 78°C, FABMS 333 (M⁺+H), ¹H NMR (200MHz, acetone-d₆) δ 6.85-6.65 (3H, m, H-2',5',6'), 6.44 (2H, s, H-5,6), 5.25 (1H, q, H-3), 5.08 (1H, d, *J*=5.8Hz, H-2), 2.91 (1H, dd, *J*=5.2 and 16Hz, H-4eq), 2.77 (1H, dd, *J*=6.3 and 16.5Hz, H-4ax), 1.92 (3H, s, -CH₃).
**4. (-)-3-O-butyryl mesquitol (4b):** m.p 80°C, FABMS 383 (M⁺+23), ¹H NMR (200MHz, acetone-d₆) δ 6.65-6.84 (3H, m, H-2',5',6'), 6.42 (2H, s, H-5,6), 5.25 (1H, q, H-3), 5.02 (1H, d, *J*=6.3 Hz, H-2), 3.02 (1H, dd, *J*=5.2 and 16.4Hz, H-4eq), 2.82 (1H, dd, *J=*7 and 16.3Hz, H-4ax), 2.15 (2H, t, H-2"), 1.46 (2H, m, H-3"), 0.78 (3H, t, H-4'').
**5. (-)-3-O-hexanoyl mesquitol (4c)**: m.p 81°C, FABMS 389 (M⁺+H), ¹H NMR (200MHz, acetone-d₆) δ 6.84-6.65 (3H, m, H-2',5',6'), 6.42 (2H, s, H-5,6), 5.24 (1H, q, H-3), 5.02 (1H, d, *J*=6.5Hz, H-2), 2.97 (1H, dd, *J*=5.2 and 16.4Hz, H-4eq), 2.78 (1H, dd, *J*=7 and 16.3Hz, H-4ax), 2.15 (2H, t, H-2"), 1.45 (6H, m, H-3"-5"), 0.78 (3H, t, H-6").
**6. (-)-3-O-Decanoyl mesquitol (4d):** m.p 84°C, FABMS 467 (M⁺+23), ¹H NMR (200MHz, acetone-d₆) δ 6.85-6.65 (3H, m, H-2',5',6'), 6.45 (1H, d, *J*=8Hz, H-5), 6.35 (1H, d, *J*=8 Hz, H-6), 5.23 (1H, q, H-3), 5.02 (1H, d, *J*=7.0Hz, H-4), 2.95 (1H, dd, *J*=5.2 and 16.3Hz, H-4eq), 2.75 (1H, dd, *J*= 7 and 16.3Hz, H-4), 2.15(2H, t, H-2"), 1.46 (2H, m, H-3"), 1.20 (12H, brs, H-4"-9"), 0.85 (3H, t, H-10").
**7. (-)-3-O-myristoyl mesquitol (4e):** m.p 83°C, FABMS 501 (M⁺+H), ¹H NMR (200MHz, acetone-d₆) δ 6.85-6.65 (3H, m, H-2',5',6'), 6.45 (1H, d, *J*=8Hz, H-5), 6.35 (1H, d, *J*=8Hz, H-6) 5.23 (1H, q, H-3), 5.01 (1H, d, *J*=7.0Hz, H-4), 2.95 (1H, dd, *J*=5.2 and 16.3Hz, H-4eq), 2.75 (1H, dd, *J*=7 and 16.3Hz, H-4), 2.16 (2H, t, H-2"), 1.45 (2H, m, H-3"), 1.20 (20H, brs, H-4"-13"), 0.85 (3H, t, H-14").
**8. (-)-3-O-Palmitoyl mesquitol (4f):** m.p 87°C, FABMS 529 (M⁺+H), ¹H NMR (200MHz, acetone-d₆) δ 6.87-6.72 (3H, m, H-2',5',6'), 6.55 (1H, d, *J*=8Hz, H-5), 6.45 (1H, d, *J*=8Hz, H-6), 5.30 (1H, q, H-3), 5.02 (1H, d, *J*=7.5Hz, H-2), 2.98 (1H, dd, J= 5.2 and 16.3Hz, H-4eq), 2.80 (1H, dd, *J*=7 and 16.3Hz, H-4ax), 2.18 (2H, t, H-2"), 1.45 (2H, m, H-3"), 1.25 (24H, brs, H-4"-15"), 0.89 (3H, t, H-16").
**9. (-)-3-O-Steroyl mesquitol (4g):** m.p 90°C, FABMS 557 (M⁺+H), ¹H NMR (200MHz, acetone-d₆) δ 6.85-6.70 (3H, m, H-2',5',6'), 6.50 (1H, d, *J*=8Hz, H-5), 6.40 (1H, d, *J*=8Hz, H-6), 5.28 (1H, q, H-3), 5.01 (1H, d, *J*=7.5Hz, H-2), 3.01 (1H, dd, *J*=5.2 and 16.3 Hz, H-4eq), 2.83 (1H, dd, *J*=7 and 16.3Hz, H-4ax), 2.17 (2H, t, H-2"), 1.47 (2H, m, H-3"), 1.23 (28H, brs, H-4"-17"), 0.90 (3H, t, H-18").
**10.(-)-3-O-benzoyl mesquitol (4h).** m.p 248°C, FABMS 395 (M⁺+H), ¹H NMR. (200MHz, acetone-d₆) δ 7.92 (2H, m, H-2",6"), 7.60-7.46 (3H, m, H-3",4",5"), 6.95 (1H, brs, H-2'), 6.81 (2H, m, H-5',6'), 6.45 (2H, s, H-5,6), 5.50 (1H, q, H-3), 5.25 (1H, d, *J*=6.7Hz, H-2), 3.10 (1H, dd, *J*=5.2 and 16.0Hz, H-4eq), 2.95 (1H, dd, *J*=7.5 and 16Hz, H-4ax).
**11. (-)-3-O-(o-chloro benzoyl) mesquitol (4i):** m.p 252°C, FABMS 429 (M⁺+H), ¹H NMR (200MHz, acetone-d₆) δ 7.85 (1H, d, *J*=8Hz, H-6"), 7.45 (1H, m, H-3"), 7.36 (2H, m, H-4",5"), 6.92 (1H, s, H-2'), 6.80 (2H, m, H-5',6'), 6.42 (1H, brs, H-5), 5.70 (1H, brs, H-6), 5.46 (1H, q, H-3), 5.21 (1H, d, *J*=7.2Hz, H-2), 3.10 (1H, dd, *J*=6.0 and 16.2Hz, H-4eq), 2.90 (1H, dd, *J*=8.0 and 16.2Hz, H-4ax).
**12. (-)-3-O-(*p*-methoxy benzoyl) mesquitol (4j):** m.p 254°C, FABMS 425 (M⁺+H), ¹H NMR (200MHz, acetone-d₆) δ 7.78 (2H, d, *J*=8Hz, H-2",6"), 6.82 (2H, d, *J=*8Hz, H-3",5"), 6.75 (3H, m, H-2',5',6'), 6.52 (1H, d, *J*=8Hz, H-5) 6.42 (1H, d, *J*=8Hz H-6), 5.45 (1H, q, H-3), 5.15 (1H, d, *J*=6.5Hz, H-2), 3.80 (3H, s, -OMe), 3.05 (1H, dd, *J*=5.2 and 16.0Hz, H-4eq), 2.85 (1H, dd, *J*=7.5 and 16.0Hz, H-4ax).
**13. (-)-3-O-(*p*-flouro benzoyl) mesquitol (4k):** m.p 263°C, FABMS 413 (M⁺+H), ¹H NMR (200MHz, acetone-d₆) δ 7.95 (2H, m, H-3",5"), 7.22 (2H, m, H-2",6"), 6.93 (1H, s, H-2'), 6.80 (2H, m, H-5',6'), 6.44 (2H, s, H-5,6), 5.48 (1H, q, H-3), 5.22 (1H, d, *J*=6.5Hz, H-2), 3.15 (1H, dd, *J*= 5.2 and 16.0Hz, H-4eq), 2.96 (1H, dd, *J*= 7.5 and 16.0 Hz, H-4ax).

### Brief description of the accompanied drawings:

The invention is illustrated by following accompanying drawings wherein:

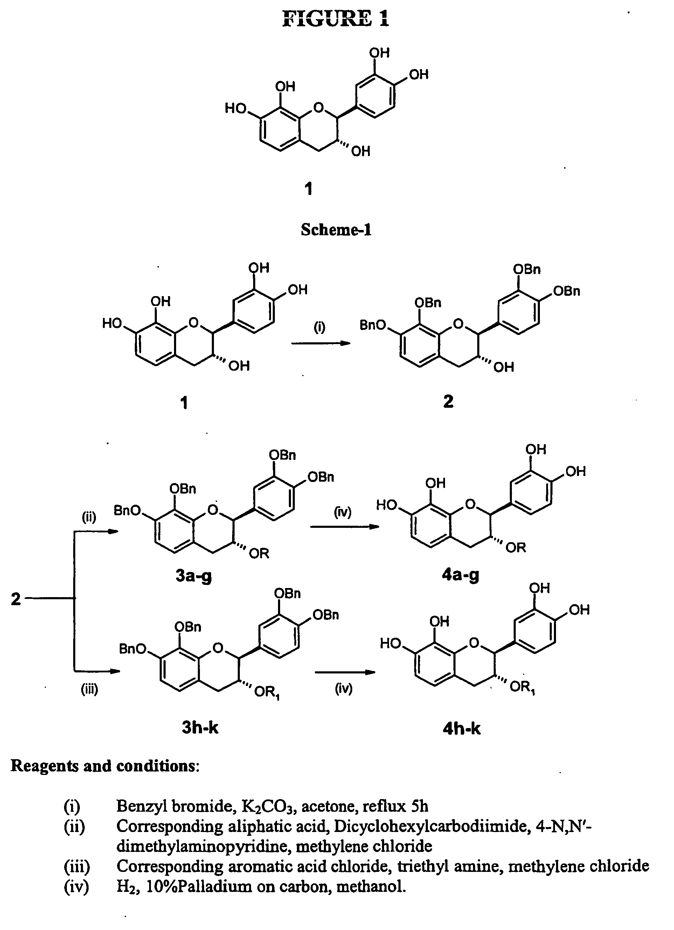

**Fig.1** represent the structure of (-)-mesquitol.

**Scheme-1:** represents synthetic routes for preparing aliphatic esters **(4a-g)** and aromatic esters **(4h-k)** of (-)-mesquitol.

**Table.1** is a representation depicting the α-glucosidase inhibitory activities (IC₅₀ values) of compounds **(4a-k).**

**Table.1**

| **Compound** | **IC₅₀ (µM)** |
|---|---|
| 1 | 82.32 |
| 4a | 46.31 |
| 4b | 46.18 |
| 4c | 49.34 |
| 4d | 13.46 |
| 4e | 12.56 |
| 4f | 9.56 |
| 4g | 54.45 |
| 4h | 77.76 |
| 41 | 43.26 |
| 4j | 67.26 |
| 4k | 32.82 |
| 1-deoxy nojirimycin | 50 |

## Claims

1. Mesquitol and its semi-synthetic 3- O-alkyl or aryl esters represented by the general formula (4) 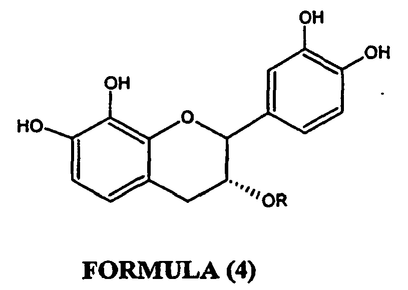 wherein, R = H, CₙH₂ₙ₊₁ (n = 1 to 16); -COC₆H₄X [wherein X = H, F, Cl, Br, I, NO₂, CN, NH₂, OR₁ {R₁ = H; CₙH₂ₙ₊₁ (n = 1 to 8)}].

2. Compounds of claim 1, represented by general formula (4), 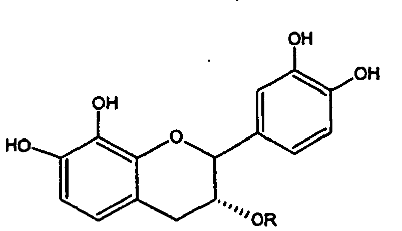 where R is selected from the following table:
| **Serial No.** | **Compound Code** | **R** |
|---|---|---|
| 1. | 4a | Acetyl |
| 2. | 4b | Butyryl |
| 3. | 4c | Hexanoyl |
| 4. | 4d | Decanoyl |
| 5. | 4e | Myristoyl |
| 6. | 4f | Palmitoyl |
| 7. | 4g | Stearoyl |
| 8. | 4h | Benzoyl |
| 9. | 4i | o-chloro benzoyl |
| 10. | 4j | p-methoxy benzoyl |
| 11. | 4k | p-flouro benzoyl |

3. Compounds of claims 1 and 2 which exhibit α-glucosidase inhibitory activity.

4. Compounds of claim 3, wherein the α-glucosidase inhibitory activity of 3-O-aliphatic esters of (-) mesquitol increases with an increase in carbon chain length up to sixteen carbon atoms.

5. Compounds of claim 2, wherein palmitoyl, myristoyl and decanoyl esters of (-) mesquitol are more potent α-glucosidase inhibitors than the standard drug 1-deoxy nojirimycin.

6. Compounds of claim 3, wherein the α-glucosidase inhibitory activity of 3-O-aromatic esters of (-) mesquitol is better than that of the parent compound.

7. Compounds of claim 2, wherein the benzoyl and p-fluorobenzyl ester of (-) mesquitol are more potent α-glucosidase inhibitors than the standard drug 1- deoxy nojirimycin.

8. Compounds of claim 1, which are useful in the management and treatment of diseases like hyperglycemia, hyper insulinemia, hypolipoproteinemia, cancer, viral infection, hepatitis B and C, HIV and AIDS.

9. Compounds of claim 1, which have an IC₅₀ value in the range of 32.0 to 83.0 µm.

10. A composition with α-glucosidase inhibitor activity, wherein the composition comprises a pharmaceutically effective dosage of (-) mesquitol or its analogues or combination thereof and optionally pharmaceutically acceptable additives and is administered to the subject in need of.

11. A composition of claim 10, wherein the composition is used singly or in combination with pharmaceutically acceptable analogues.

12. A composition of claim 10, wherein the composition may be administered systemically or orally.

13. A composition of claim 10, wherein the subject is selected from animals or mammals, preferably humans.

14. A process for the preparation of semi-synthetic 3-O-alkyl or aryl esters of (-) mesquitol, the said process comprising the steps of:
a) drying the wood of Dichrostachys cinerea in shade,
b) powdering the shade dried wood of step (a),
c) extracting the powder of step (b) with petroleum ether, followed by halogenated hydrocarbon solvent to obtain the plant extracts and a plant residue,
d) soaking the plant residue of step (c) in methanol, filtering and concentrating the methanol extract to obtain a residue,
e) purifying the residue of step (d) over silica gel column by eluting with mixture of organic solvents,
f) obtaining pure (-) mesquitol,
g) adding to (-) mesquitol of step (f) anhydrous potassium carbonate, a ketonic solvent, benzyl halide and refluxing the mixture under nitrogen atmosphere for a time period of 2hours to 8hours,
h) filtering the mixture of step (g), washing the residue with ketonic solvent, combining the filtrate and the washings evaporating under reduced pressure to obtain a residue,
i) purifying the residue of step (h) to obtain pure tetra-O-benzyl-mesquitol of formula (2),
j) treating the compound of formula (2) of step (i) with N,N'-Dicyclohexyl carbodiimide (DCC) aliphatic acid in an anhydrous methylene chloride under nitrogen atmosphere followed by addition of 4-dimethyl aminopyridine, stirring the mixture for 6h to 18 h at room temperature, filtering the mixture, washing the residue with methylene chloride, combining the filtrate and washing to obtain a methylene chloride solution,
k) washing the methylene chloride solution of step (j) with water, drying over anhydrous sodium sulphate, filtering and evaporating the solvent to obtain a residue,
l) purifying the residue of step (k) to obtain the required 3-O-alkyl esters of tetra-O-benzyl (-) mesquitol of formulae 3a to 3g.
m) providing a cooled solution of compound (2) in anhydrous methylene chloride along with triethyl amine, flushing nitrogen,
n) adding required benzoyl chloride to the mixture of step (m) and stirring the mixture for 2h to 6h at an ambient temperature,
o) adding water to the reaction mixture of step (n), extracting with methylene chloride, separating methylene chloride layer and aqueous layer,
p) drying the methylene chloride layer of step (o) over anhydrous sodium sulphate, filtering and evaporating the solvent to obtain a residue,
q) purifying the residue of step (p) to obtain 3-O-aryl ester of tetra-O-benzyl (-) mesquitol of formula 3h to 3k,
r) stirring the compound of step (1) or step (q) in an alcoholic solution in the presence of palladium charcoal and hydrogen for a time period of 4h to 8h at room temperature, and
s) filtering the mixture of step (r), removing the solvent from the filtrate to obtain the required 3-O-alkyl or aryl esters of (-) mesquitol of general formula (4).

15. A process of claim 14, wherein in step (e) the mixture of organic solvent used for eluting, is a mixture of chloroform and methanol.

16. A process of claim 14, wherein the mixture of solvents used is chloroform-methanol (96:4).

17. A process of claim 14, wherein the ketonic solvent used is selected from ethyl-methyl ketone, acetone or methyl isobutyl ketone.

18. A process of claim 17, wherein the ketonic solvent used is acetone.

19. A process of claim 14, wherein in step (j) the aliphatic acid used is selected from a group consisting of acetic acid, butyric acid, hexanoic acid, decanoic acid, myristic acid, palmitic acid or stearic acid.

20. A process of claim 14, wherein in step (n) the benzoyl chloride used is selected from a group consisting of halo benzoyl chloride, alkoxy benzoyl chloride, cyano benzoyl chloride, amino benzoyl chloride, or nitro benzoyl chloride.

21. A process of claim 20, wherein the halo benzoyl chloride used is o-chloro benzoyl chloride or p-fluoro benzoyl chloride.

22. A process of claim 20, wherein the alkoxy benzoyl chloride used is p-methoxy benzoyl chloride.

23. A process of claim 14, wherein in step (s) the 3-O-esters of (-) mesquitol obtained exhibit α-glucosidase inhibitor activity.

24. A process of claim 23, wherein the α-glucosidase inhibitory activity of 3-O-aliphatic esters of (-) mesquitol increases with an increase in carbon chain length up to sixteen carbon atoms.

25. A process of claim 14, wherein palmitoyl, myristoyl and decanoyl esters of (-) mesquitol are more potent than the standard α-glucosidase inhibitor 1-deoxy nojirimycin.

26. A process of claim 25, wherein the α-glucosidase inhibitory activity of 3-O-aromatic esters of (-) mesquitol is better than that of the parent compound.

27. A process of claim 25, wherein the 3-O aromatic esters of (-) mesquitol are benzoyl, o-chlorobenzoyl, p-methoxy benzoyl or p-fluoro benzoyl esters.

28. A process of claim 14, wherein the benzoyl and p-fluorobenzyl esters of (-) mesquitol are more potent than the standard α-glucosidase inhibitor 1- deoxy nojirimycin.

29. A process of claim 14, wherein the 3-O-alkyl or aryl esters of (-) mesquitol obtained are useful in the management and treatment of diseases like hyperglycemia, hyper insulinemia, hypolipoproteinemia, cancer, viral infection, hepatitis B and C, HIV and AIDS.

30. A process of claim 14, wherein the 3-O-alkyl or aryl esters of (-) mesquitol obtained have an IC₅₀ value in the range of 32.0 to 83.0 µm.

## Patentansprüche

1. Mesquitol und dessen halb-synthetische 3-O-Alkyl- oder Arylester, dargestellt durch die allgemeine Formel (4) 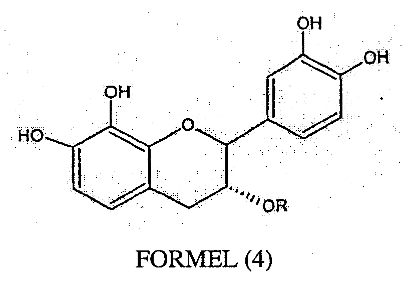 wobei R = H, CₙH₂ₙ₊₁ (n = 1 bis 16); -COC₆H₄X [wobei X = H, F, Cl, Br, I, NO₂, CN, NH₂, OR₁ {R₁ = H; CₙH₂ₙ₊₁ (n = 1 bis 8)}].

2. Verbindungen nach Anspruch 1, dargestellt durch die allgemeine Formel (4), 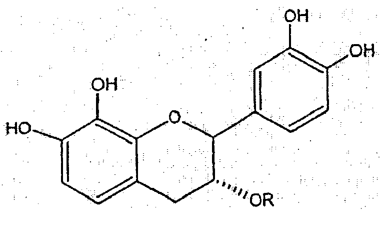 wobei R aus der folgenden Tabelle ausgewählt ist:
| **Laufende Nr.** | **Verbindungs-Code** | **R** |
|---|---|---|
| 1. | 4a | Acetyl |
| 2. | 4b | Butyryl |
| 3. | 4c | Hexanoyl |
| 4. | 4d | Decanoyl |
| 5. | 4e | Myristoyl |
| 6. | 4f | Palmitoyl |
| 7. | 4g | Stearoyl |
| 8. | 4h | Benzoyl |
| 9. | 4i | o-Chlorbenzoyl |
| 10. | 4j | p-Methoxybenzoyl |
| 11. | 4k | p-Fluorbenzoyl |

3. Verbindungen nach den Ansprüchen 1 und 2, welche α-Glucosidase hemmende Aktivität aufweisen.

4. Verbindungen nach Anspruch 3, wobei die α-Glucosidase hemmende Aktivität von 3-O-aliphatischen Estern von (-)Mesquitol mit einem Anstieg der Kohlenstoffkettenlänge auf bis zu 16 Kohlenstoffatome ansteigt.

5. Verbindungen nach Anspruch 2, wobei Palmitoyl-, Myristoyl- und Decanoylester von (-)Mesquitol wirksamere α-Glucosidaseinhibitoren sind als der standardmäßige Arzneistoff 1-Deoxynojirimycin.

6. Verbindungen nach Anspruch 3, wobei die α-Glucosidase hemmende Aktivität von 3-O-aromatischen Estern von (-)Mesquitol besser ist als die der Stammverbindung.

7. Verbindungen nach Anspruch 2, wobei die Benzoyl- und p-Fluorbenzylester von (-)Mesquitol wirksamere α-Glucosidaseinhibitoren sind als der standardmäßige Arzneistoff 1-Deoxynojirimycin.

8. Verbindungen nach Anspruch 1, welche nützlich in der Therapie und der Behandlung von Krankheiten wie Hyperglykämie, Hyperinsulinämie, Hypolipoproteinämie, Krebs, Virusinfektion, Hepatitis B und C, HIV und AIDS sind.

9. Verbindungen nach Anspruch 1, welche einen IC₅₀-Wert im Bereich von 32,0 bis 83,0 µm aufweisen.

10. Zusammensetzung mit α-Glucosidaseinhibitor-Aktivität, wobei die Zusammensetzung eine pharmazeutisch wirksame Menge von (-)Mesquitol oder dessen Analoga oder Kombination davon und gegebenenfalls pharmazeutisch verträgliche Additive umfasst und an einen Patienten, der diese benötigt, verabreicht wird.

11. Zusammensetzung nach Anspruch 10, wobei die Zusammensetzung allein oder in Kombination mit pharmazeutisch verträglichen Analoga verwendet wird.

12. Zusammensetzung nach Anspruch 10, wobei die Zusammensetzung systemisch oder oral verabreicht werden kann.

13. Zusammensetzung nach Anspruch 10, wobei der Patient ausgewählt ist aus Tieren oder Säugern, vorzugsweise Menschen.

14. Verfahren zur Herstellung von halb-synthetischen 3-O-Alkyl- oder Arylestern von (-)Mesquitol, wobei das Verfahren die Schritte umfasst:
a) Trocknen des Holzes von Dichrostachys cinerea im Schatten,
b) Pulverisieren des im Schatten getrockneten Holzes aus Schritt (a),
c) Extrahieren des Pulvers aus Schritt (b) mit Petrolether, gefolgt von halogeniertem Kohlenwasserstofflösungsmittel, um die Pflanzenextrakte und einen Pflanzenrückstand zu erhalten,
d) Einweichen der Pflanzenrückstände aus Schritt (c) in Methanol, Filtern und Konzentrieren des Methanolextrakts, um einen Rückstand zu erhalten,
e) Reinigen des Rückstandes aus Schritt (d) über einer Kieselgelsäule durch Elution mit einem Gemisch aus organischen Lösungsmitteln,
f) Erhalten von reneim (-) Mesquitol,
g) Zufügen von wasserfreiem Kaliumcarbonat, einem ketonischen Lösungsmittel, Benzylhalogenid zum (-)Mesquitol aus Schritt (f) und Rückflusskochen des Gemisches unter Stickstoffatmosphäre für einen Zeitraum von 2 bis 8 Stunden,
h) Filtern des Gemisches aus Schritt (g), Waschen des Rückstandes mit ketonischem Lösungsmittel, Kombinieren des Filtrats und der Waschlösung, Eindampfen unter verringertem Druck, um einen Rückstand zu erhalten,
i) Reinigen des Rückstandes aus Schritt (h), um reines Tetra-O-benzylmesquitol der Formel (2) zu erhalten,
j) Behandeln der Verbindung der Formel (2) aus Schritt (i) mit N,N'-Dicyclohexylcarbodiimid (DCC) aliphatische Säure, in einem wasserfreien Methylenchlorid unter Stickstoffatmosphäre, gefolgt von der Zugabe von 4-Dimethylaminopyridin, Rühren des Gemisches für 6 bis 18 Stunden bei Raumtemperatur, Filtern des Gemisches, Waschen des Rückstandes mit Methylenchlorid, Kombinieren des Filtrats und der Waschlösung, um eine Methylenchloridlösung zu erhalten,
k) Waschen der Methylenchloridlösung aus Schritt (j) mit Wasser, Trocknen über wasserfreiem Natriumsulphat, Filtrieren und Eindampfen des Lösungsmittels, um einen Rückstand zu erhalten,
l) Reinigen des Rückstandes aus Schritt (k), um die benötigten 3-O-Alkylester von Tetra-O-benzyl(-)mesquitol der Formeln 3a bis 3g zu erhalten,
m) Bereitstellen einer gekühlten Lösung von Verbindung (2) in wasserfreiem Methylenchlorid zusammen mit Triethylamin, Stickstoffspülen,
n) Zufügen von benötigtem Benzoylchlorid zu dem Gemisch aus Schritt (m) und Rühren des Gemisches für 2 bis 6 Stunden bei Raumtemperatur,
o) Zufügen von Wasser zum Reaktionsgemisch aus Schritt (n), Extrahieren mit Methylenchlorid, Trennen der Methylenchloridschicht und der wässrigen Schicht,
p) Trocknen der Methylenchloridschicht aus Schritt (o) über wasserfreiem Natriumsulphat, Filtrieren und Eindampfen des Lösungsmittels, um einen Rückstand zu erhalten,
q) Reinigen des Rückstandes aus Schritt (p), um 3-O-Arylester von Tetra-O-benzyl(-)mesquitol der Formel 3h bis 3k zu erhalten,
r) Rühren der Verbindung aus Schritt (1) oder Schritt (q) in einer alkoholischen Lösung in Gegenwart von Palladiumholzkohle und Wasserstoff über einen Zeitraum von 4 bis 8 Stunden bei Raumtemperatur, und
s) Filtrieren des Gemisches aus Schritt (r), Entfernen des Lösungsmittels aus dem Filtrat, um die benötigten 3-O-Alkyl- oder Arylester von (-)Mesquitol der allgemeinen Formel (4) zu erhalten.

15. Verfahren nach Anspruch 14, wobei in Schritt (e) das zum Verdünnen verwendete Gemisch aus organischen Lösungsmitteln ein Gemisch aus Chloroform und Methanol ist.

16. Verfahren nach Anspruch 14, wobei das verwendete Gemisch aus Lösungsmitteln Chloroform-Methanol (96:4) ist.

17. Verfahren nach Anspruch 14, wobei das verwendete ketonische Lösungsmittel ausgewählt ist aus Ethylmethylketon, Aceton oder Methylisobutylketon.

18. Verfahren nach Anspruch 17, wobei das verwendete ketonische Lösungsmittel Aceton ist.

19. Verfahren nach Anspruch 14, wobei in Schritt (j) die verwendete aliphatische Säure aus Essigsäure, Buttersäure, Hexansäure, Decansäure, Myristinsäure, Palmitinsäure oder Stearinsäure ausgewählt ist.

20. Verfahren nach Anspruch 14, wobei in Schritt (n) das verwendete Benzoylchlorid ausgewählt ist aus Halogenbenzoylchlorid, Alkoxybenzoylchlorid, Cyanobenzoylchlorid, Aminobenzoylchlorid oder Nitrobenzoylchlorid.

21. Verfahren nach Anspruch 20, wobei das verwendete Halogenbenzoylchlorid o-Chlorbenzoylchlorid oder p-Fluorbenzoylchlorid ist.

22. Verfahren nach Anspruch 20, wobei das verwendete Alkoxybenzoylchlorid p-Methoxybenzoylchlorid ist.

23. Verfahren nach Anspruch 14, wobei in Schritt (s) die erhaltenen 3-O-Ester von (-)Mesquitol α-Glucosidaseinhibitoraktivität aufweisen.

24. Verfahren nach Anspruch 23, wobei die α-Glucosidase hemmende Aktivität von 3-O-aliphatischen Estern von (-)Mesquitol mit einem Anstieg der Kohlenstoffkettenlänge auf bis zu 16 Kohlenstoffatome ansteigt.

25. Verfahren nach Anspruch 14, wobei Palmitoyl-, Myristoyl- und Decanoylester von (-)Mesquitol wirksamer sind als der standardmäßige α-Glucosidaseinhibitor 1-Deoxynojirimycin.

26. Verfahren nach Anspruch 25, wobei die α-Glucosidase hemmende Aktivität von 3-O-aromatischen Estern von (-)Mesquitol besser ist als die der Stammverbindung.

27. Verfahren nach Anspruch 25, wobei die 3-O-aromatischen Ester von (-)Mesquitol Benzoyl, o-Chlorbenzoyl, p-Methoxybenzoyl oder p-Fluorbenzoylester sind.

28. Verfahren nach Anspruch 14, wobei die Benzoyl- und p-Fluorbenzylester von (-)Mesquitol wirksamer sind als der standardmäßige α-Glucosidaseinhibitor 1-Deoxynojirimycin.

29. Verfahren nach Anspruch 14, wobei die erhaltenen 3-O-Alkyl- oder Arylester des (-)Mesquitols in der Therapie und der Behandlung von Krankheiten wie Hyperglykämie, Hyperinsulinämie, Hypolipoproteinämie, Krebs, Virusinfektion, Hepatitis B und C, HIV und AIDS nützlich sind.

30. Verfahren nach Anspruch 14, wobei die erhaltenen 3-O-Alkyl- oder Arylester des (-)Mesquitols einen IC₅₀-Wert im Bereich von 32,0 bis 83,0 µm aufweisen.

## Revendications

1. Mesquitol et ses 3-O-alkyl ou aryl esters semi-synthétiques représentés par la formule générale (4) 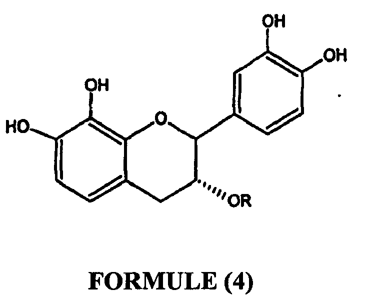 où R = H, CₙH₂ₙ₊₁ (n = 1 à 16) ; -COC₆H₄X [où X = H, F, Cl, Br, I, NO₂, CN, NH₂, OR₁ {R₁ = H ; CₙH₂ₙ₊₁ (n = 1 à 8)}].

2. Composés selon la revendication 1 représentés par la formule générale (4) 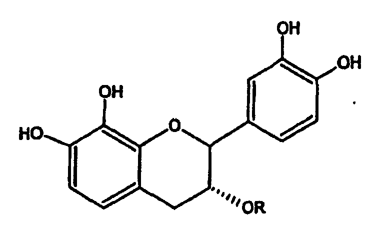 où R est choisi dans le tableau suivant :
| **n°. d'ordre** | **code de composé** | **R** |
|---|---|---|
| 1. | 4a | acétyle |
| 2. | 4b | butyryle |
| 3. | 4c | hexanoyle |
| 4. | 4d | décanoyle |
| 5. | 4e | myristoyle |
| 6. | 4f | palmitoyle |
| 7. | 4g | stéaroyle |
| 8. | 4h | benzoyle |
| 9. | 4i | o-chloro benzoyle |
| 10. | 4j | p-méthoxy benzoyle |
| 11. | 4k | p-fluoro benzoyle |

3. Composés selon les revendications 1 et 2 qui présentent une activité d'inhibiteur d'α-glucosidase.

4. Composés selon la revendication 3 où l'activité d'inhibiteur d'α-glucosidase des 3-O-esters aliphatiques de (-) mesquitol augmente avec une augmentation de la longueur de la chaîne carbonée jusqu'à seize atomes de carbone.

5. Composés selon la revendication 2 où les palmitoyl, myristoyl et décanoyl esters du (-) mesquitol sont des inhibiteurs d'α-glucosidase plus actifs que le médicament standard 1-désoxy nojirimycine.

6. Composés selon la revendication 3 où l'activité d'inhibiteur d'α-glucosidase des 3-O-esters aromatiques de (-) mesquitol est meilleure que celle du composé mère.

7. Composés selon la revendication 2 où le benzoyl et le p-fluorobenzyl ester de (-) mesquitol sont des inhibiteurs d'α-glucosidase plus actifs que le médicament standard 1-désoxy nojirimycine.

8. Composés selon la revendication 1 qui sont utiles dans la prise en charge et le traitement de maladies comme l'hyperglycémie, l'hyper insulinémie, l'hypolipoprotéinémie, le cancer, une infection virale, l'hépatite B et C, VIH et le SIDA.

9. Composés selon la revendication 1 qui ont une valeur CI₅₀ dans la plage de 32,0 à 83,0 µm.

10. Composition à activité d'inhibiteur d'α-glucosidase, où la composition comprend un dosage pharmaceutiquement efficace de (-) mesquitol ou de ses analogues ou d'une combinaison de ceux-ci et éventuellement des additifs pharmaceutiquement acceptables et est administrée au sujet qui en a besoin.

11. Composition selon la revendication 10 où la composition est utilisée seule ou en combinaison avec des analogues pharmaceutiquement acceptables.

12. Composition selon la revendication 10, où la composition peut être administrée par voie systémique ou orale.

13. Composition selon la revendication 10 où le sujet est choisi parmi les animaux ou les mammifères, de préférence les humains.

14. Procédé pour la préparation de 3-O-alkyl ou aryl esters semi-synthétiques de (-) mesquitol, ledit procédé comprenant les étapes de :
a) sécher du bois de Dichrostachys cinerea à l'ombre,
b) réduire en poudre le bois séché à l'ombre de l'étape (a),
c) extraire la poudre de l'étape (b) avec de l'éther de pétrole, puis un solvant hydrocarboné halogéné pour obtenir les extraits végétaux et un résidu végétal,
d) tremper le résidu végétal de l'étape (c) dans le méthanol, filtrer et concentrer l'extrait au méthanol pour obtenir un résidu,
e) purifier le résidu de l'étape (d) sur une colonne de gel de silice par élution avec un mélange de solvants organiques,
f) obtenir le (-) mesquitol pur,
g) ajouter au (-) mesquitol de l'étape (f) du carbonate de potassium anhydre, un solvant cétonique, un halogénure de benzyle et chauffer le mélange à reflux sous une atmosphère d'azote pendant une durée de 2 heures à 8 heures,
h) filtrer le mélange de l'étape (g), laver le résidu avec un solvant cétonique, combiner le filtrat et les produits de lavage, évaporer sous pression réduite pour obtenir un résidu,
i) purifier le résidu de l'étape (h) pour obtenir du tétra-O-benzyl-mesquitol pur de formule (2),
j) traiter le composé de formule (2) de l'étape (i) avec le N,N'-dicyclohexylcarbodiimide (DCC), un acide aliphatique dans un chlorure de méthylène anhydre sous une atmosphère d'azote puis addition de 4-diméthylaminopyridine, agiter le mélange pendant 6 h à 18 h à la température ambiante, filtrer le mélange, laver le résidu avec du chlorure de méthylène, combiner le filtrat et le produit de lavage pour obtenir une solution dans le chlorure de méthylène,
k) laver la solution dans le chlorure de méthylène de l'étape (j) avec de l'eau, sécher sur sulfate de sodium anhydre, filtrer et évaporer le solvant pour obtenir un résidu,
l) purifier le résidu de l'étape (k) pour obtenir les 3-O-alkyl esters de tétra-o-benzyl (-) mesquitol des formules 3a à 3g requis,
m) fournir une solution refroidie du composé (2) dans le chlorure de méthylène anhydre en même temps que de la triéthylamine, balayer avec de l'azote,
n) ajouter le chlorure de benzoyle requis au mélange de l'étape (m) et agiter le mélange pendant 2 h à 6 h à la température ambiante,
o) ajouter de l'eau au mélange réactionnel de l'étape (n), extraire avec du chlorure de méthylène, séparer la couche de chlorure de méthylène et la couche aqueuse,
p) sécher la couche de chlorure de méthylène de l'étape (o) sur sulfate de sodium anhydre, filtrer et évaporer le solvant pour obtenir un résidu,
q) purifier le résidu de l'étape (p) pour obtenir un 3-O-aryl ester de tétra-O-benzyl (-) mesquitol de formule 3h à 3k,
r) agiter le composé de l'étape (1) ou de l'étape (q) dans une solution alcoolique en présence de palladium charbon et d'hydrogène pendant une durée de 4h à 8h à la température ambiante, et
s) filtrer le mélange de l'étape (r), retirer le solvant du filtrat pour obtenir les 3-O-alkyl ou aryl esters de (-) mesquitol de formule générale (4) requis.

15. Procédé selon la revendication 14 où, dans l'étape e), le mélange de solvants organiques utilisé pour l'élution est un mélange de chloroforme et de méthanol.

16. Procédé selon la revendication 14 où le mélange de solvants utilisé est du chloroforme-méthanol (96 : 4).

17. Procédé selon la revendication 14 où le solvant cétonique utilisé est choisi parmi l'éthylméthylcétone, l'acétone ou la méthylisobutylcétone.

18. Procédé selon la revendication 17 où le solvant cétonique utilisé est l'acétone.

19. Procédé selon la revendication 14 où, dans l'étape (j), l'acide aliphatique utilisé est choisi dans un groupe consistant en l'acide acétique, l'acide butyrique, l'acide hexanoïque, l'acide décanoïque, l'acide myristique, l'acide palmitique ou l'acide stéarique.

20. Procédé selon la revendication 14 où, dans l'étape (n), le chlorure de benzoyle utilisé est choisi dans un groupe consistant en chlorure d'halogénobenzoyle, chlorure d'alcoxybenzoyle, chlorure de cyanobenzoyle, chlorure d'aminobenzoyle ou chlorure de nitrobenzoyle.

21. Procédé selon la revendication 20 où le chlorure d'halogénobenzoyle utilisé est le chlorure de o-chlorobenzoyle ou le chlorure de p-fluorobenzoyle.

22. Procédé selon la revendication 20 où le chlorure d'alcoxybenzoyle utilisé est le chlorure de p-méthoxybenzoyle.

23. Procédé selon la revendication 14 où, dans l'étape (s), les 3-O-esters de (-) mesquitol obtenus présentent une activité d'inhibiteur d'α-glucosidase.

24. Procédé selon la revendication 23 où l'activité d'inhibiteur d'α-glucosidase des 3-O-esters aliphatiques de (-) mesquitol augmente avec une augmentation de la longueur de la chaîne carbonée jusqu'à seize atomes de carbone.

25. Procédé selon la revendication 14 où les palmitoyl, myristoyl et décanoyl esters de (-) mesquitol sont plus actifs que l'inhibiteur d'α-glucosidase standard 1-désoxy nojirimycine.

26. Procédé selon la revendication 25 où l'activité d'inhibiteur d'α-glucosidase des 3-O-esters aromatiques de (-) mesquitol est meilleure que celle du composé mère.

27. Procédé selon la revendication 25 où les 3-O-esters aromatiques de (-) mesquitol sont des benzoyl, o-chlorobenzoyl, p-méthoxybenzoyl ou p-fluorobenzoyl esters.

28. Procédé selon la revendication 14 où les benzoyl et p-fluorobenzyl esters de (-) mesquitol sont plus actifs que l'inhibiteur d'α-glucosidase standard 1-déroxy nojirimycine.

29. Procédé selon la revendication 14 où les 3-O-alkyl ou aryl esters de (-) mesquitol obtenus sont utiles dans la prise en charge et le traitement de maladies comme l'hyperglycémie, l'hyper insulinémie, l'hypolipoprotéinémie, le cancer, une infection virale, l'hépatite B et C, VIH et le SIDA.

30. Procédé selon la revendication 14 où les 3-O-alkyl ou aryl esters de (-) mesquitol obtenus ont une valeur CI₅₀ dans la plage de 32,0 à 83,0 µm.
